# EUROPEAN PATENT APPLICATION

(11) **EP 1 872 721 A1**
(43) Date of publication of application: **02.01.2008**
(21) Application number: 06731317.1
(22) Date of filing: 06.04.2006
(51) Int. Cl.: A61B 6/03, A61B 5/055

(54) **IMAGE DISPLAY DEVICE**

(30) Priority: 07.04.2005 JP 2005110666
(71) Applicant: Nemoto Kyorindo Co., Ltd., Tokyo 113-0033 (JP)
(72) Inventor: NEMOTO, Shigeru c/o Nemoto Kyorindo Co., Ltd.,, Tokyo 1130033 (JP)
(74) Representative: Cabinet Plasseraud
(86) International application number: PCT/JP2006/307369
(87) International publication number: WO 2006/109692

(57) **Abstract**

There is provided an image display device capable of easily checking an enormous number of tomograms. Display combining means (326) synthesizes continuous n tomograms from an enormous number of tomograms and causes image displaying means (323) to display the synthesized image. Thus, the number of tomograms to be displayed becomes 1/n. Thus, it is possible to easily check the enormous number of tomograms of series data. Moreover, since no thinning of tomograms is performed, it is possible to prevent oversight of necessary information.

## Description

### Technical Field

The present invention relates to an image display device for displaying tomographic images, and more particularly, to an image display device capable of switching a number of displayed successive tomographic images as series data.

### Background Art

Presently available imaging apparatuses in medical facilities for capturing a number of successive tomographic images of a human body include CT (Computed Tomography) scanners and MRI (Magnetic Resonance Imaging) apparatuses. Various types of image display devices have been developed for displaying the tomographic images provided by those apparatuses.

Such an image display device is realized, for example, by installing dedicated application software on a personal computer and allows a plurality of successive tomographic images stored as series data to be shown in order on a display. In a typical personal computer, however, an input operation is performed through a keyboard including entry keys such as alphabetical keys and a mouse, so that the general versatility is excellent but the operability is poor.

To address this, an image display device proposed by the present applicant has a dedicated switching operation means which has a to-and-fro operation member slidable forward and rearward. In the image display device, when the to-and-fro operation member is slid forward or rearward, tomographic images shown on a display are accordingly switched to allow an operator to easily view a number of successive tomographic images.

Particularly, in the abovementioned image display device, slightly sliding the to-and-fro operation member from the initial position switches the displayed tomographic images at a low speed, while largely sliding the to-and-fro operation member switches the displayed tomographic images at a high speed. The operator can intuitively adjust the switching speed of the tomographic images flexibly (See, for example, Patent Documents 1 to 4).
Patent Document 1: Japanese Patent Laid-Open No. 2003-084910
Patent Document 2: Japanese Patent Laid-Open No. 2003-132766
Patent Document 3: Japanese Patent Laid-Open No. 2003-150138
Patent Document 4: Japanese Patent Laid-Open No. 2003-339695

In the currently used imaging apparatuses such as CT scanners, more and more tomographic images have been taken in the same succession direction to increase the density of imaging, so that a vast number of tomographic images are provided for the image display device. Even when tomographic images are displayed at a variable switching speed in the image display device, it is not easy to see the vast number of tomographic images of series data. One conceivable solution thereof is to allow switching of tomographic images at an extremely high speed, but this approach increases the possibility of overlooking necessary information.

For example, when a linear lesion exists in the body of a patient, the lesion is linearly displayed in tomographic images and is thus easily found if the longitudinal direction of the lesion is parallel with the surface direction of the tomographic images. However, if the longitudinal direction of the lesion intersects with the surface direction of the tomographic images, the lesion is displayed as dot(s) in the tomographic images, and is thus unlikely to be found.

### Disclosure of the Invention

The present invention has been made in view of the abovementioned problems, and it is an object thereof to provide an image display device which allows an operator to easily check a number of successive tomographic images.

The image display device according to an aspect of the present invention includes a tomographic image storing means, a tomographic image displaying means, a switching operation means, an image switching means, and a display combining means. The tomographic image storing means stores series data formed of a number of successive tomographic images. The tomographic image displaying means displays the stored tomographic images. The switching operation means receives an input operation to switch the tomographic images in a continuous forward or rearward direction. The image switching means switches the displayed tomographic images in the successive forward or rearward direction in accordance with the input operation. The display combining means combines n successive images of a number of tomographic images, and causes the tomographic image displaying means to display the combined image. As a result, the number of tomographic images is reduced by a factor of n.

Various means referred to in the present invention may be arranged to perform their functions, and may comprise dedicated hardware for performing a predetermined function, a data processing apparatus whose predetermined function is given by a computer program, a predetermined function performed in a data processing apparatus according to a computer program, or a combination thereof.

Various components referred to in the present invention do not need to be a separate entity. A plurality of components may be constructed as one member, a certain component may be part of another component, or a certain component may have a portion overlapping a portion of another component.

### Effect of the Invention

According to the image display device of the present invention, n successive images are combined, and the number of displayed tomographic images is reduced by a factor of n. This allows easy check of a number of the tomographic images of the series data. In addition, in the present invention the tomographic images are "combined", no tomographic image is omitted, so that overlooking necessary information can be prevented.

### Brief Description of the Drawings

Fig. 1 is a schematic diagram showing the logical configuration of a tomography system according to an embodiment of the present invention;
Fig. 2 is a perspective view showing the outer appearance of the tomography system;
Fig. 3 is a perspective view showing the outer appearance of an image display device;
Fig. 4 is a plan view showing the outer appearance of a control unit;
Fig. 5 is a block diagram showing the circuit configuration of the image display device;
Fig. 6 is a side view showing the internal structure of an input operation apparatus;
Fig. 7 is an exploded perspective view showing the assembly of the main parts of the input operation apparatus;
Fig. 8 is a perspective view showing the structure of the main part of the input operation apparatus;
Figs. 9(a) to 9(d) are diagrams showing the operation steps of the input operation apparatus;
Fig. 10 is a schematic front view showing a tomographic image and the like displayed on a display unit:
Fig. 11 is a schematic front view showing a tomographic image and the like displayed on the display unit:
Fig. 12 is a schematic diagram showing a plurality of successive tomographic images;
Fig. 13 is a schematic diagram showing a combined image of a plurality of tomographic images;
Fig. 14 is a flow chart showing an early stage of the processing operation of the image display device;
Fig. 15 is a flow chart showing the processing operation for combining tomographic images by the image display device;
Fig. 16 is a flow chart showing the processing operation for switching displayed tomographic images by the image display device;
Fig. 17 is a schematic diagram showing an exemplary display in a first modification; and
Fig. 18 is a schematic diagram showing an exemplary display in a second modification.

### Description of Reference Numerals

- 100: INPUT OPERATION APPARATUS SERVING AS SWITCHING OPERATION MEANS AND SHOT INPUTTING MEANS
- 204: FUNCTION SELECTING SWITCH SERVING AS FUNCTION SELECTING MEANS
- 300: IMAGE DISPLAY DEVICE
- 322: TOMOGRAPHIC IMAGE STORING MEANS
- 323: TOMOGRAPHIC IMAGE DISPLAYING MEANS
- 324: IMAGE SWITCHING MEANS
- 326: DISPLAY COMBINING MEANS
- 331: PART EXTRACTING MEANS
- 332: ORTHOGONAL IMAGE PRODUCING MEANS
- 333: ORTHOGONAL IMAGE STORING MEANS
- 334: ORTHOGONAL IMAGE DISPLAYING MEANS
- 336: INDEX IMAGE PRODUCING MEANS
- 337: INDEX IMAGE DISPLAYING MEANS

### Best Mode for Carrying Out the Invention

### [Configuration of Embodiment]

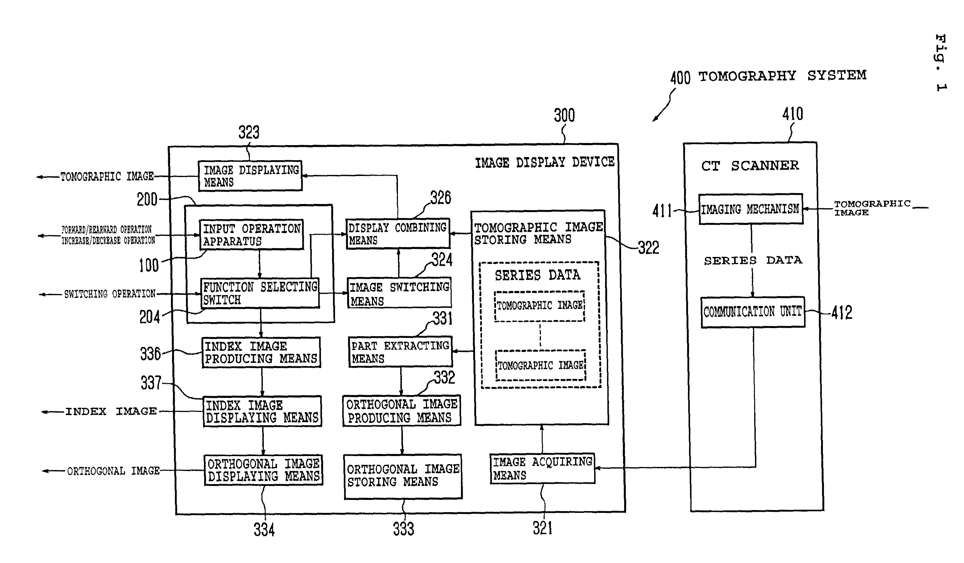

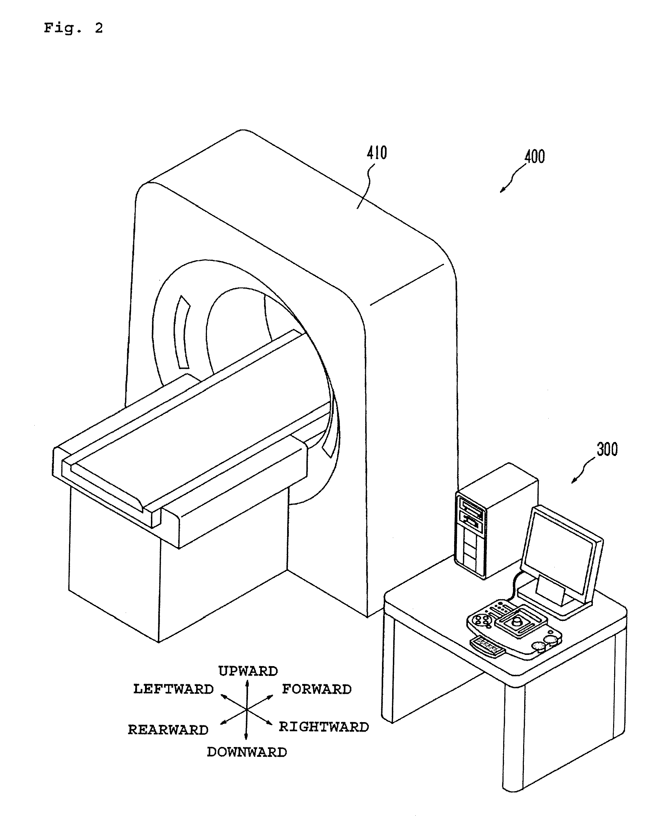

An embodiment of the present invention will hereinafter be described with reference to Figs. 1 to 18. As shown in Figs. 1 and 2, image display device 300 of the embodiment is formed as part of tomography system 400 and is online connected to CT scanner 410 serving as an imaging diagnostic apparatus.

CT scanner 410 includes imaging mechanism 411 serving as an imaging means and communication unit 412 serving as an image supply means. Imaging mechanism 411 takes a number of successive tomographic images from a patient (not shown). A plurality of tomographic images successively taken from the patient at predetermined intervals are accumulated as series data. Communication unit 412 transmits the data to image display device 300.

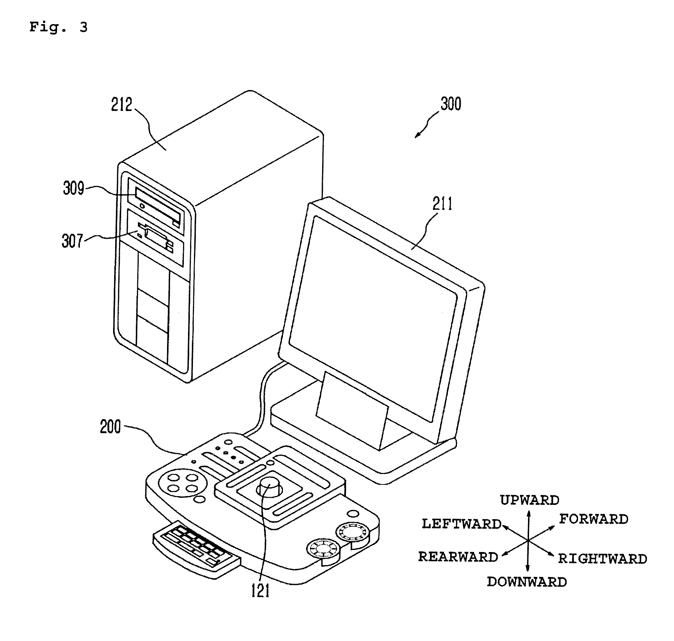

As shown in Fig. 3, image display device 300 of the embodiment includes control unit 200, display unit 211, body unit 212 and the like as main components. Control unit 200 and display unit 211 are connected to body unit 212.

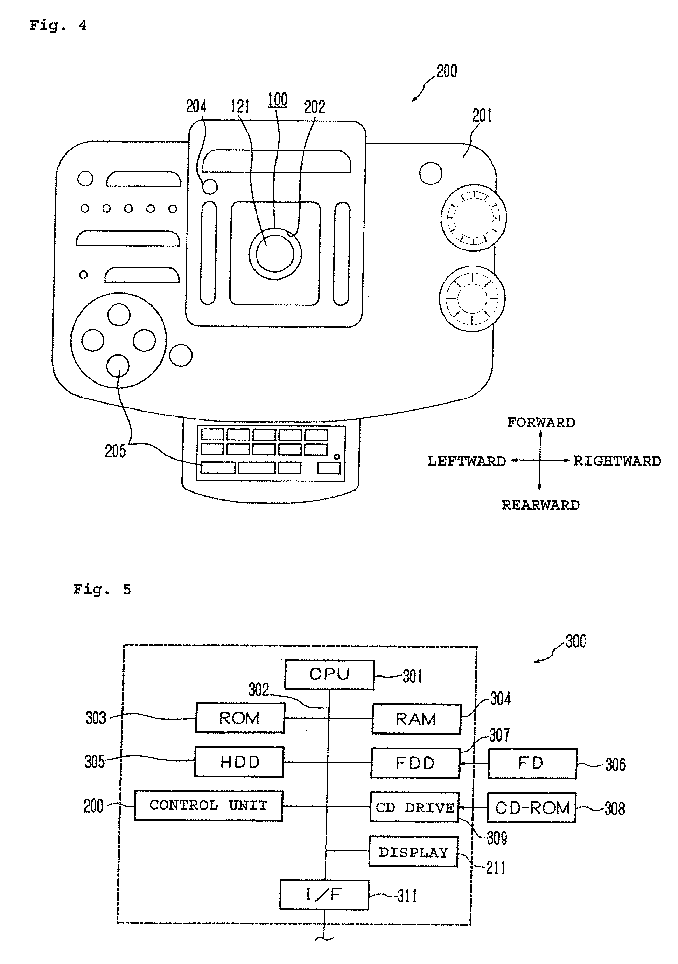

As shown in Fig. 4, control unit 200 includes housing 201 which has through hole 202 formed at the center of the upper surface thereof. Joystick 121 of input operation apparatus 100 serving as a switching operation means and a shot inputting means is exposed upward from the through hole 202.

Function selecting switch 204 serving as a function selecting means is placed at front of joystick 121 on the left. Although described later in detail, function selecting switch 204 is used to switch the functions of input operation apparatus 100. Control unit 200 has various operation keys 205 mounted thereon in addition to input operation apparatus 100 and function selecting switch 204 described above. Operation keys 205 have various functions associated therewith.

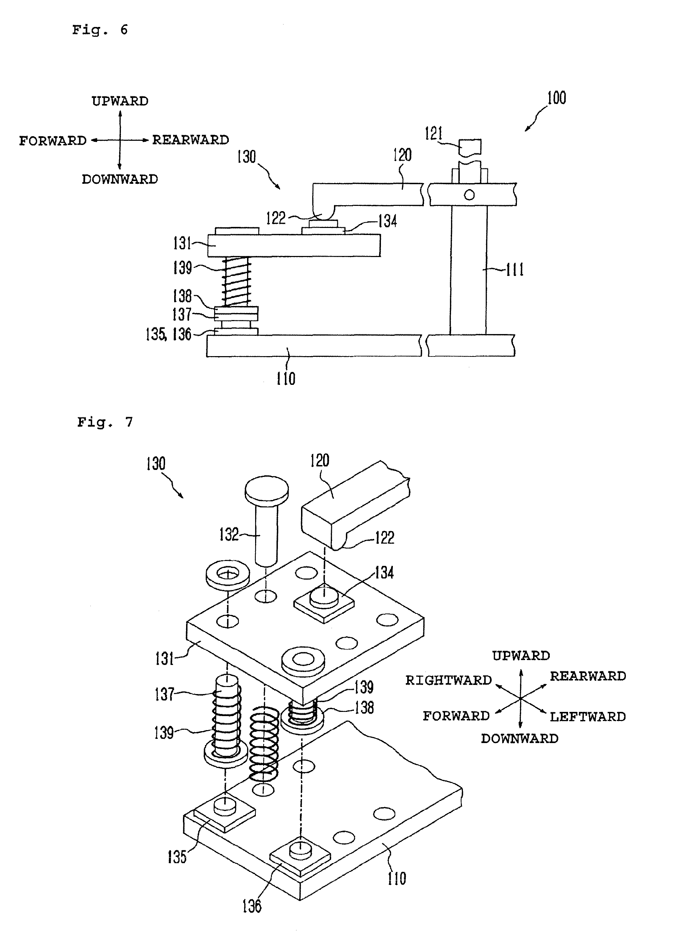

As shown in Figs. 6 to 9, input operation apparatus 100 includes planar base member 110 elongated in a to-and-fro direction and to-and-fro operation member 120. To-and-fro support mechanisms 111 provided to stand on the right and left at the center of base member 110 support the member 120 serving as a second operation member to be pivotable forward and rearward on the center.

Joystick 121 is provided to stand at the center of the upper surface of to-and-fro operation member 120 and protrudes upward from through hole 202 in control unit 200 as shown in Figs. 3 and 4. Each of end portions 122 in the front and rear of to-and-fro operation member 120 is formed in a semi-cylindrical shape protruding downward. Multi-stage detectors 130 are formed as a front switch and a rear switch at the positions where they are pushed by end portions 122 in the front and rear from above.

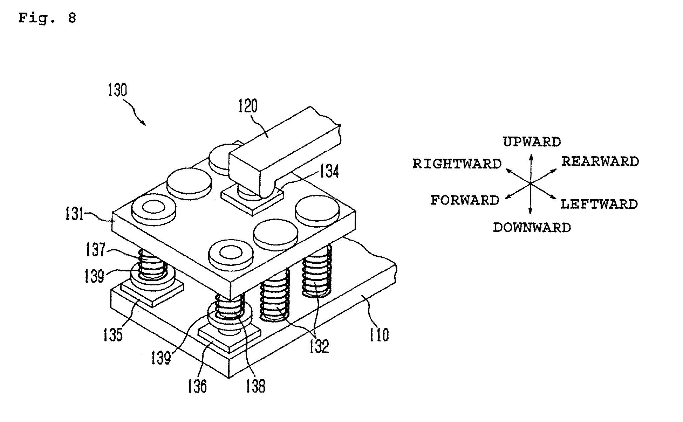

Multi-stage detector 130 has planar and rectangular up-and-down operation member 131 to which end portion 122 of to-and-fro operation member 120 is opposed from above. As shown in Figs. 7 and 8, two pairs of guide shafts 132 as operation support members are provided to stand on the upper surface of based member 110 in the front and rear end portions. Guide shafts 132 support up-and-down operation member 131 to be slidable upward and downward.

A coil spring (not shown) is wound on each guide shaft 132 and serves as an operation biasing mechanism to elastically bias up-and-down operation member 131 upward. Since each guide shaft 132 is formed as a stopper having an upper end extended outward, up-and-down operation member 131 is held at the positions of the upper ends of guide shafts 132 such that it is biased upward and is slidable upward and downward.

Two press-contact detection elements 135 and 136 are placed in the front and rear ends on the upper surface of base member 110 such that one of them is located on the left and the other on the right. Two displacement press members 137 and 138 are slidably mounted on up-and-down operation member 131 at the positions where they are opposed to press-contact detection elements 135 and 136 from above, respectively.

Coil spring 139 is wound on each of displacement press members 137 and 138 and serves as a press biasing mechanism to bias displacement press members 137 and 138 downward against up-and-down operation member 131. Since each of displacement press members 137 and 138 is formed as a stopper having an upper end extend outward, their lower ends are opposed to press-contact detection elements 135 and 136 while they are biased downward and slidable upward and downward.

In multi-stage detector 130 of the embodiment, the vertical lengths of two displacement press members 137 and 138 that protrude downward from up-and-down operation member 131 are different from each other in steps, so that the distances between displacement press members 137 and 138 and opposed press-contact detection elements 135 and 136 are different. In multi-stage detector 130 of the embodiment, third press-contact detection element 134 is placed on the upper surface of up-and-down operation member 131, and end portion 122 of to-and-fro operation member 120 is opposed to press-contact detection element 134 from above.

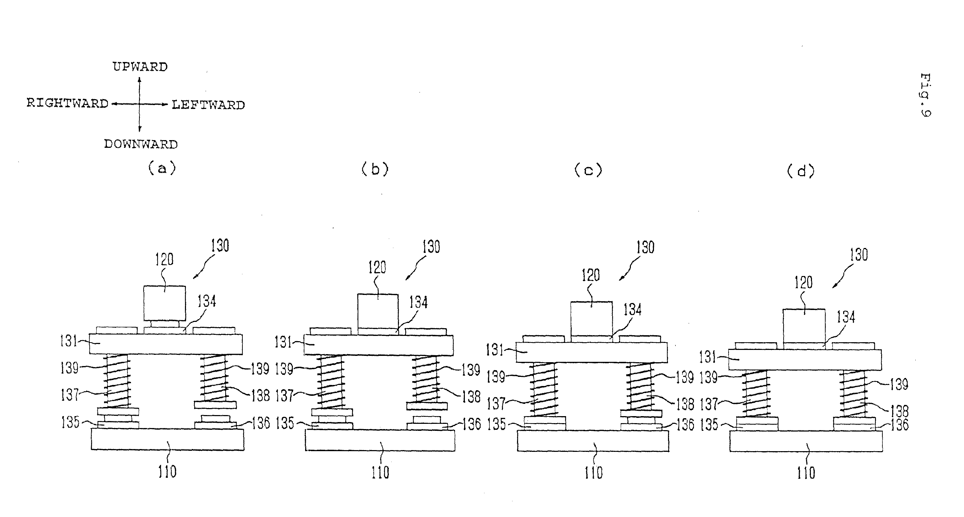

Each of press-contact detection elements 134 to 136 is formed such that element displacement member 142 is supported to be elastically retractable on detection element body 141. Press-contact detection elements 134 to 136 require the same pressure for operation. Up-and-down operation member 131 is biased upward by the coil spring as described above, so that a downward displacement of end portion 122 of to-and-fro operation member 120 first presses only the element 134 on the upper surface of up-and-down operation member 131 as shown in Figs. 9(a) and 9(b).

When end portion 122 of to-and-fro operation member 120 is further displaced downward, longer displacement press member 137 presses press-contact detection element 135 on the upper surface of base member 110 as shown in Fig. 9(c). When end portion 122 of to-and-fro operation member 120 is further displaced downward, shorter displacement press member 138 presses the other press-contact detection element 136 as shown in Fig. 9(d).

As shown in Fig. 5, image display device 300 of the embodiment includes CPU (Central Processing Unit) 301 as hardware which is a main part of a computer. CPU 301 is connected through bus line 302 to pieces of hardware such as ROM (Read Only Memory) 303, RAM (Random Access Memory) 304, HDD 305 (Hard Disc Drive) 305, FDD (Flexible Disc Drive) 307 on which FD (Flexible Disc-cartridge) 306 is replaceably loaded, CD (Compact Disc) drive 309 on which CD-ROM 308 is replaceably loaded, control unit 200, display unit 211, and I/F (Interface) unit 311.

In image display device 300 of the embodiment, pieces of hardware such as ROM 303, RAM 304, HDD 305, replaceable FD 306, and replaceable CD-ROM 308 correspond to information storage media, and computer programs or resources for CPU 301 are stored as software on at least one of them. Such software is previously installed on image display device 300 and is read as data by CPU 301 when image display device 300 is started up.

CPU reads the data of the appropriate computer program to execute various processing, to cause image display device 300 of the embodiment to logically have various means such as image acquiring means 321, tomographic image storing means 322, tomographic image displaying means 323, image switching means 324, display combining means 326, part extracting means 331, orthogonal image producing means 332, orthogonal image storing means 333, orthogonal image displaying means 334, index image producing means 336, and index image displaying means 337, as shown in Fig. 1.

Image acquiring means 321 corresponds to the function of CPU 301 which recognizes data received by I/F unit 311 in accordance with the computer program stored on RAM 304 and acquires the series data from CT scanner 410. Tomographic image storing means 322 corresponds to the storage area created on HDD 305 to be recognized by CPU 301 in accordance with the abovementioned computer program and stores the series data acquired by image acquiring means 321.

Tomographic image displaying means 323 corresponds to the function of CPU 301 which displays on display unit 211 the data stored on HDD 305 and displays the tomographic images of the series data stored in tomographic image storing means 322. Image switching means 324 corresponds to the function of CPU 301 which recognizes an operation signal from control unit 200 to perform predetermined data processing and switches the tomographic images displayed by tomographic image displaying means 323 in accordance with an input operation on to-and-fro operation member 120 of control unit 200.

More specifically, in image display device 300 of the embodiment, when joystick 121 of input operation apparatus 100 is inclined forward at a predetermined angle with a predetermined pressure to press only press-contact detection element 134 on the upper surface of up-and-down operation member 131 of multi-stage detector 130 in the front, only one tomographic image of the series data displayed is switched forward in response. When joystick 121 is inclined rearward to press only press-contact detection element 134 of multi-stage detector 130 in the rear, only one tomographic image is switched rearward in response.

Then, when joystick 121 is further inclined forward or rearward to cause longer displacement press member 137 of multi-stage detector 130 in the front or rear to press press-contact detection element 135 on the upper surface of base member 110, the displayed tomographic images of the series data are successively switched at a low speed forward or rearward.

Then, when joystick 121 is further inclined forward or rearward to cause shorter displacement press member 138 of multi-stage detector 130 in the front or rear to press the other press-contact detection element 136, the displayed tomographic images of the series data are successively switched at a high speed forward or rearward.

Display combining means 326 corresponds to the function of CPU 301 which performs predetermined data processing on a plurality of data stored on HDD 305. When the number of images to be combined is entered through input operation apparatus 100, display combining means 326 combines successive tomographic images by the number, and provides the combined image as a tomographic image for display by tomographic image displaying means 323.

More specifically, in image display device 300 of the embodiment, the abovementioned forward or rearward switching of tomographic images and the increase/decrease of the number of images to be combined through input operation apparatus 100 are selectively performed in response to an operation on function selecting switch 204. When the increase/decrease of the number of tomographic images to be combined is selected with function selecting switch 204, a forward inclination of joystick 121 of input operation apparatus 100 to a first stage increases the number of images to be combined by one, while a rearward inclination of joystick 121 to the first stage reduces the number of images to be combined by one.

A forward or rearward inclination of joystick 121 to a second stage increases or reduces the number of images to be combined at a low speed. A forward or rearward inclination of joystick 121 to a third stage increases or reduces the number of images to be combined at a high speed. The combination of a plurality of tomographic images is performed, for example, by superimposing one image on another.

Part extracting means 331 and orthogonal image producing means 332 correspond to the function of CPU 301 which performs predetermined data processing with a plurality of data stored on HDD 305. Part extracting means 331 extracts linear parts at the same position from a number of the successive tomographic images. Orthogonal image producing means 332 places the linear parts extracted by part extracting means 331 in the succession direction of the tomographic images, to produce an orthogonal image corresponding to a tomographic image in a direction orthogonal to the succession direction of the tomographic images.

Orthogonal image storing means 333 also corresponds to the storage area created on HDD 305 to be recognized by CPU 301, and stores the orthogonal images produced by orthogonal image producing means 332. Orthogonal image displaying means 334 corresponds to the function of CPU 301 which displays on display unit 211 the data stored on HDD 305, and displays the stored orthogonal images.

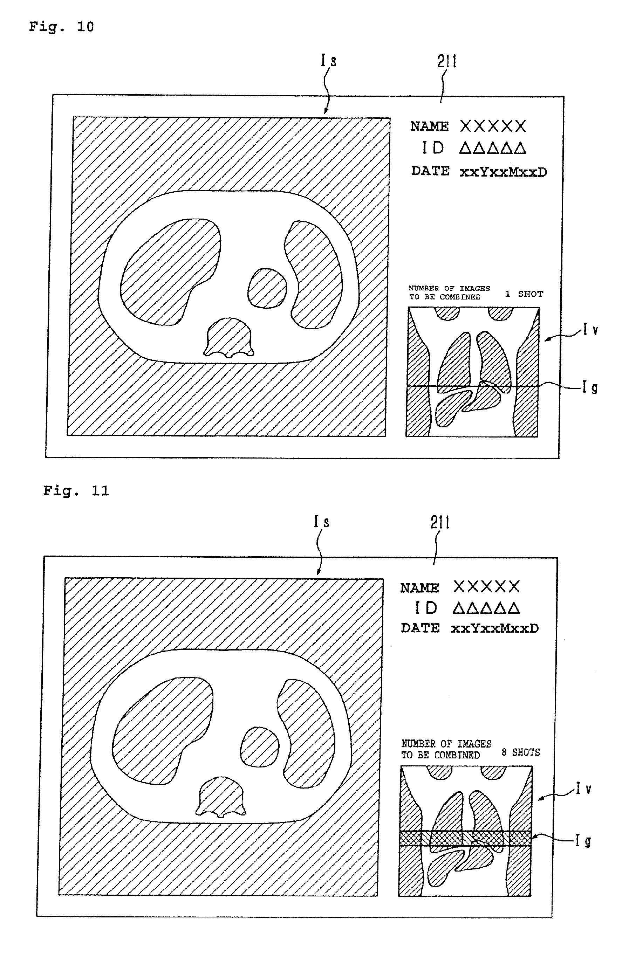

More specifically, when tomographic images arranged in parallel with XY directions and continued in a Z direction have x, y dots in the XY directions and continue up to z shots, a linear part of y dots in the Y direction is extracted at the position of x/2 dots in the X direction from each of the z images, and the z linear parts are placed in the succession direction of the tomographic images. This results in an orthogonal image corresponding to a tomographic image in parallel with YZ directions. For example, as shown in Fig. 10, orthogonal image Iv is displayed together with tomographic image Is on display unit 211.

Index image producing means 336 corresponds to the function of CPU 301 which performs predetermined data processing. When the number of images to be combined is entered through input operation apparatus 100 as described above, index image producing means 336 produces an index image with a layer thickness corresponding to the number of images to be combined. Index image displaying means 337 corresponds to the function of CPU 301 which displays the processed data on display unit 211 and displays index image Ig produced by index image producing means 336 such that the index image is superimposed on orthogonal image Iv at the position associated with displayed combined tomographic image Is, as shown in Figs. 10 and 11.

Although the abovementioned various means of image display device 300 are accomplished by pieces of hardware such as HDD 305 and I/F unit 311 as required, they are mainly implemented by CPU 301 as a piece of hardware functioning in accordance with the computer programs stored on the information storage medium such as RAM 304.

The computer programs are stored on the information storage medium such as RAM 304 as software for causing CPU 301 or the like to perform, for example, the reception of the series data by I/F unit 311 from CT scanner 410, the storage of the received series data on HDD 305, the display of the tomographic images of the stored series data on display unit 211, the forward or rearward switching of the displayed tomographic images at the variable speed in accordance with the input operation on input operation apparatus 100 of control unit 200, the combination of the entered number of tomographic images shown on display unit 211 into one in accordance with the input operation on input operation apparatus 100, the extraction of the linear parts at the same position from the successive tomographic images, the production of the orthogonal image by placing the extracted linear parts in the succession direction of the tomographic images, the storage of the produced orthogonal image on HDD 305, the display of the stored orthogonal image on display unit 211, the production of the index image with the layer thickness corresponding to the number of images to be combined entered through input operation apparatus 100, and the display of the produced index image such that it is superimposed on the orthogonal image at the position associated with the displayed combined tomographic image.

### [Operation of the Embodiment]

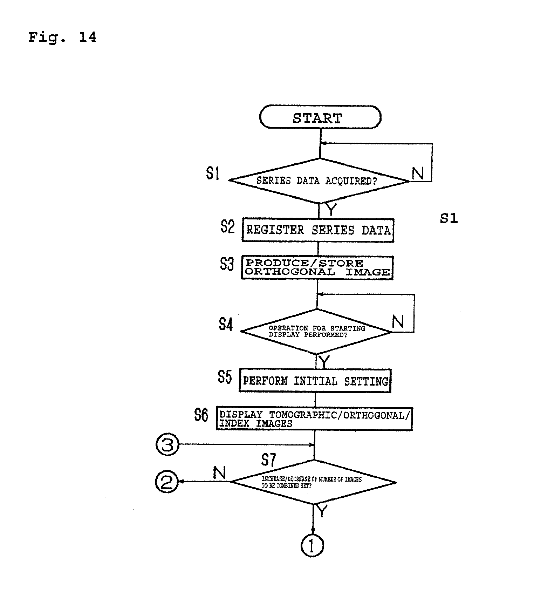

In the abovementioned configuration, the test method in tomography system 400 of the embodiment will hereinafter be described in order. First, a number of successive tomographic images are taken from a patient (not shown) by CT scanner 410 and are registered as series data in image display device 300 as shown in Fig. 14 (steps S1 and S2).

After the series data are registered in image display device 300 in this manner, linear parts at the same position are extracted from the tomographic images and placed in the succession direction to automatically produce an orthogonal image corresponding to a tomographic image in a direction orthogonal to the succession direction of the tomographic images (step S3).

The produced orthogonal image is stored on HDD 305 or the like. Then, when an operator (not shown) performs a predetermined operation for starting display on control unit 200 (step S4), initial settings are performed for image display (step S5).

The initial settings are, for example, setting the number of tomographic images to be combined to one, producing an index image corresponding to the one as the number of images to be combined, displaying the first tomographic image of the series data, and superimposing the index image on the orthogonal image at the position associated with the first one of the series data.

An initial screen is displayed on display unit 211 based on the initial settings. On the initial screen, for example, the first tomographic image of the series data is shown, and the orthogonal image is shown on the lower left thereof, where the linear index image is superimposed on the orthogonal image at the uppermost position (step S6).

As shown in Fig. 10, image display device 300 of the embodiment shows the number of images to be combined as text data together with various data including the name of the series data, the ID of a patient, and the date of shooting, above the orthogonal image. Thus, the space on the display screen is used with no waste.

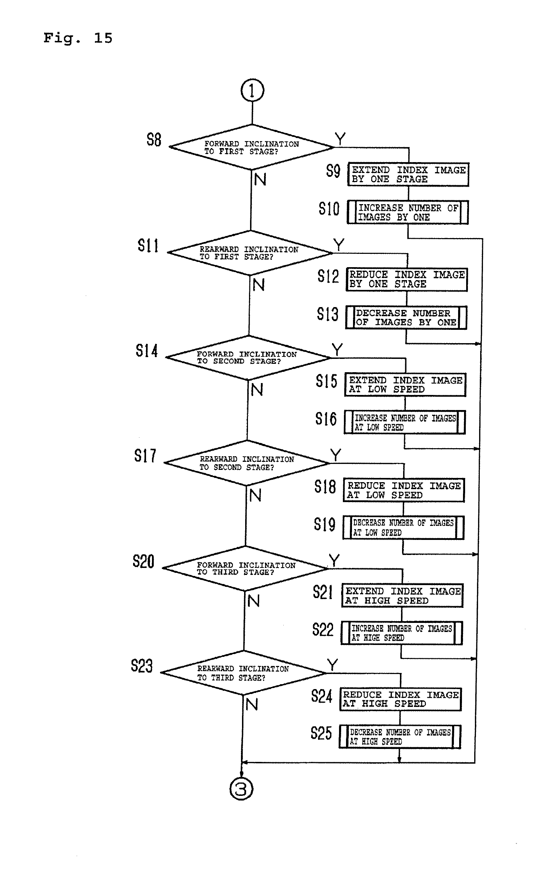

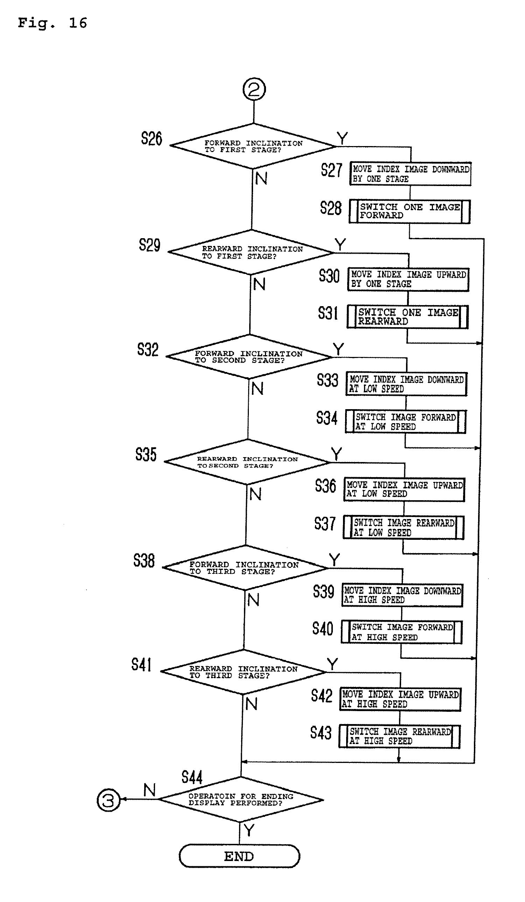

In this state, function selecting switch 204 is operated (step S7) to select the forward or rearward switching of displayed tomographic images as shown in Fig. 15 (steps S8 to S25). Alternatively, the switch 204 is operated to increase/decrease the number of displayed tomographic images to be combined as shown in Fig. 16 (steps S26 to S43) through an input operation on input operation apparatus 100.

When the increase/decrease of the number of images to be combined is selected, in case joystick 121 of input operation apparatus 100 is inclined forward to the first stage as shown in Fig. 15 (step S8), the number of tomographic images to be combined is increased by one (step S10), and accordingly the index image is extended by one stage (step S9). When each input operation is performed, the number of tomographic images to be combined is increased by one, and the index image is extended by one stage.

For example, when only the first tomographic image is shown on display unit 211, as described above, a forward inclination of joystick 121 to the first stage causes the data of the second tomographic image to be read and combined with the first tomographic image. Then the combined tomographic image is displayed. Assuming that the vertical width of the index image corresponding to the single tomographic image is 1 mm, the lower edge is extended downward by 1 mm, then the vertical width becomes 2 mm.

When joystick 121 of input operation apparatus 100 is inclined rearward to the first stage (step S11), the number of images to be combined is decreased by one (step S13), and accordingly the index image is reduced by one stage (step S12). When joystick 121 is inclined forward or rearward to the second stage (step S14, S17), the number of tomographic images to be combined is increased or decreased at a low speed (step S16, S19), and accordingly, the index image is extended or reduced at a low speed (step S15, S18). When joystick 121 is inclined forward or rearward to the third stage (step S20, S23), the number of tomographic images to be combined is increased or decreased at a high speed (step S22, S25), and accordingly, the index image is extended or reduced at a high speed (step S21, S24).

It goes without saying that the lower limit of the number of tomographic images to be combined and displayed is set to one, and if an input operation is performed for further reducing the number of images to be combined, the displayed tomographic image is not changed. It is also possible to set the upper limit, such as 10 images, of the number of tomographic images to be combined and displayed. In this case, even if an input operation is performed for increasing the number of images to be combined when the tomographic image resulting from the combination of the upper limit number of tomographic images is displayed, the tomographic image is not changed.

In image display device 300 of the embodiment, when function selecting switch 204 is operated to select the forward or rearward switching of tomographic images as shown in Fig. 14 (step S7), and then joystick 121 of input operation apparatus 100 is inclined forward or rearward to the first stage as shown in Fig. 16 (step S26, S29), the tomographic image shown on display unit 211 is switched forward or rearward to the next one (step S28, S31), and accordingly, the index image is moved downward or upward by one stage (step S27, S30).

When joystick 121 is further inclined forward or rearward to the second stage (step S32, S35), the displayed tomographic images are switched forward or rearward at a low speed (step S34, S37), and the index image is moved downward or upward at a low speed (step S33, S36). When joystick 121 is further inclined forward or rearward to the third stage (step S38, S41), the displayed tomographic images are switched forward or rearward at a high speed (step S40, S43), and the index image is moved downward or upward at a high speed (step S39, S42).

In image display device 300 of the embodiment, the display-switching of the tomographic images is performed on each combined tomographic images. For example, when the number of images to be combined is n, the n successive tomographic images are combined together, and combined image is displayed on the unit 212.

Then, when joystick 121 is inclined forward or rearward to the first stage, next n tomographic images(before or after the n tomographic images) are read as data, combined, and displayed on display unit 212. If the n combined and displayed tomographic images are switched m times, an image in the series data is move to (n x m)th image.

In this way, when joystick 121 is inclined forward or rearward to the second stage, the tomographic images are read as data, combined, and displayed in groups of n at the low speed. When joystick 121 is inclined forward or rearward to the third stage, the tomographic images are read as data, combined, and displayed in groups of n at the high speed.

### [Effect of the Embodiment]

In image display device 300 of the embodiment, a number of the tomographic images can be combined and displayed in groups of n successive images, as described above, the number of tomographic images to be displayed is reduced by a factor of n. This allows the operator to easily check a number of the tomographic images of series data. In this case the displayed tomographic image is provided by combining n successive images without omitting any tomographic images, therefore overlooking necessary information is prevented.

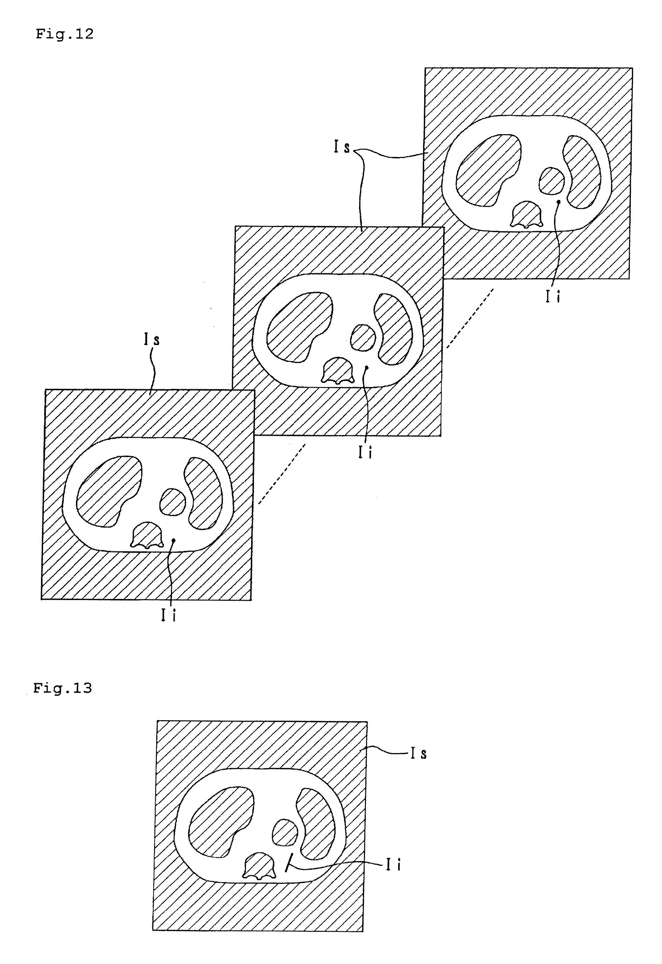

If a linear lesion exists in the body of a patient, and, the longitudinal direction of the lesion intersects with the surface direction of the tomographic images, the lesion is difficult to find since the image Ii of the lesion is displayed as dot(s) in tomographic image Is as shown in Fig. 12. Image display device 300 of the embodiment, however, combines and displays the tomographic images in groups of n successive images, so that display image Ii of the abovementioned lesion can be displayed linearly in tomographic image Is as shown in Fig. 13, to allow the operator to easily find the lesion.

The number of tomographic images to be combined can be increased or decreased arbitrarily through an input operation to flexibly adjust the speed and accuracy of check of the tomographic images. For example, the number of tomographic images to be combined can be increased to enhance the check speed at the position where any lesion is unlikely to exist, while the number of tomographic images to be combined can be decreased to improve the check accuracy at the position where any lesion is likely to exist.

The number of tomographic images to be combined can be increased or decreased without forward or rearward switching of the displayed tomographic images. When dot-like image Ii is found in tomographic image Is as described above, the number of images to be combined can be increased, to see whether or not the image corresponds to display image Ii representing the lesion.

Since the number of tomographic images to be combined can be increased or decreased at the speed associated with the input operation on input operation apparatus 100 in the plurality of steps, the number of tomographic images to be combined can be arbitrarily adjusted with ease. Particularly, the number of images to be combined can be increased or decreased at the low speed and high speed and can be increased or decreased one by one, so that a desired number of images to be combined can be set quickly and easily.

The displayed tomographic image can be switched forward or rearward at the speed associated with the input operation on input operation apparatus 100 in the plurality of steps, which enables simple and flexible display switching of tomographic images. Sine the display switching can be performed at the high speed and low speed and one by one, a desired tomographic image can be searched for and displayed quickly and easily.

The increase/decrease of the number of tomographic images to be combined and the forward/rearward switching of displayed tomographic images can be realized with one input operation apparatus 100 through the selection by function selecting switch 204. Thus, image display device 300 can be formed with a simplified structure and improved productivity.

The orthogonal image corresponding to the tomographic image in the direction orthogonal to the succession direction of the tomographic images is also displayed and the index image with the layer thickness corresponding to the number of images to be combined is superimposed on the orthogonal image at the position associated with the displayed tomographic image. As a result, it is possible to easily and reliably check the layer thickness of the tomographic images constituting the combined displayed tomographic image, and the position thereof in the whole body.

Since the linear parts at the same position are extracted from a number of the successive tomographic images and arranged to produce the orthogonal image, any orthogonal image for the series data does not need to be prepared, and the orthogonal image appropriately representing the series data can be automatically provided.

In image display device 300 of the embodiment, as shown in Fig. 10 and the like, the orthogonal image of a small size is displayed on the lower right of the tomographic image, and the text data such as the name of the patient and the number of images to be combined is shown above the orthogonal image. This allows the space on the display screen to be used with no waste.

### [Modifications of the Embodiment]

The present invention is not in any way limited to the abovementioned embodiment, but various changes and modifications may be made therein without departing from the scope of the invention. For example, in the embodiment, the orthogonal image is produced from a number of the successive tomographic images of the series data. An orthogonal image may be prepared separately for the series data.

In the above embodiment, the single input operation apparatus 100 can be used to perform the display switching of the tomographic images and the increase/decrease of the number of images to be combined through the selection by function selecting switch 204. For example, it is possible to provide two input operation apparatuses each dedicated to the display switching and the increase/decrease of the number of images to be combined.

In the above embodiment, the number of tomographic images to be combined can be increased or decreased at the high speed, low speed, and one by one with input operation apparatus 100. The increase/decrease speed may be set in various manners. For example, an input operation apparatus in an analog constructure (not shown) may be used to steplessly increase or reduce the speed.

In the above embodiment, the large rectangular tomographic image, the small rectangular orthogonal image, and the plurality of text data are placed on the rectangular display screen of display unit 211 with no wasted space. The layout on the screen may be set in various manners.

For example, as shown in Fig. 17, small orthogonal image Iv may be shown on the lower right of large tomographic image Ism resulting from combination of images, and small uncombined tomographic image I may be displayed. The uncombined tomographic image may be the first one or the one at the center of the tomographic images combined, for example. In this manner, it is possible to see the combined tomographic image and the uncombined tomographic image at the same time.

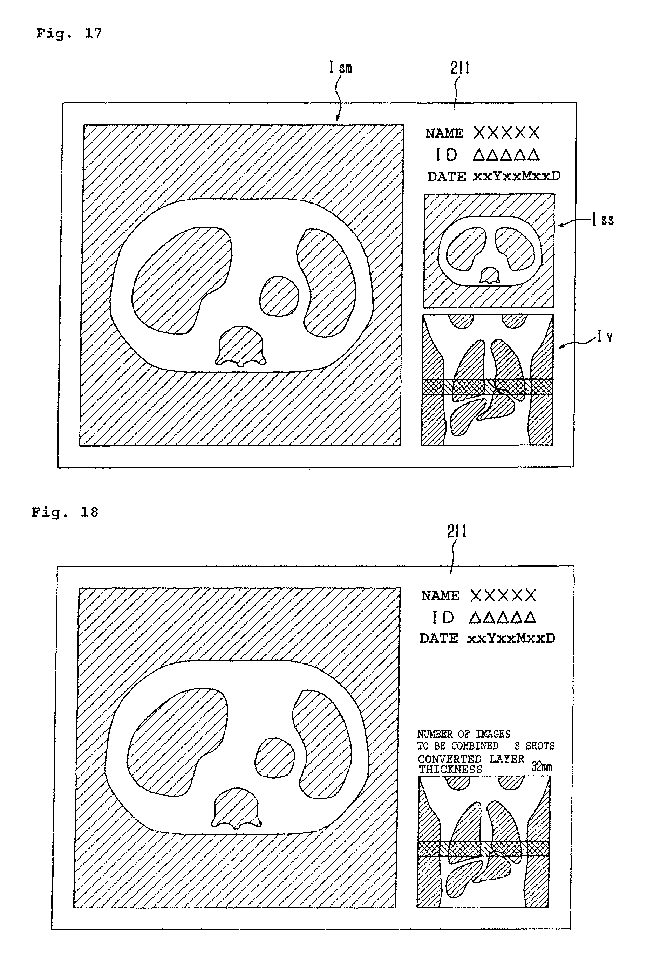

In the above embodiment, the number of images to be combined is displayed as text data on the display screen of display unit 211 for displaying the tomographic image. For example, it is possible to multiply the interval at which the tomographic images were taken from the patient by the number of images to be combined, to calculate the layer thickness of the tomographic images combined and to display the calculated layer thickness as text data as shown in Fig. 18. In this case, since not only the number of the tomographic images combined and displayed but also the layer thickness thereof can be seen, the convenience can be improved.

In the above embodiment, the plurality of tomographic images are superimposed one on another to combine the plurality of tomographic images. For example, a plurality of tomographic images may be averaged. In the above embodiment, CT scanner 410 takes the series data of the tomographic images for display on image display device 300. An MRI apparatus (not shown) may be used to take the series data.

In the above embodiment, CPU 301 operates in accordance with the computer program stored in RAM 304 or the like to realize logically various means as various functions of image display device 300. Each of the various means may be formed as specific hardware, or some of them may be stored as software in RAM 304 or the like, while others may be formed as hardware.

## Claims

1. An image display device comprising:
tomographic image storing means for storing series data formed of a number of successive tomographic images;
tomographic image displaying means for displaying the stored tomographic images;
switching operation means for receiving an input operation to switch the tomographic images in a continuous forward or rearward direction;
image switching means for switching the displayed tomographic images in the successive forward or rearward direction in accordance with the input operation; and
display combining means for combining n (where n means a natural number equal to or larger than two) successive images of the tomographic images, and for causing the tomographic image displaying means to display the combined image.

2. The image display device according to claim 1, further comprising shot inputting means for receiving an input operation for increasing or reducing the number of images to be combined,
wherein the display combining means combines the tomographic images in groups of the entered number.

3. The image display device according to claim 2, further comprising:
orthogonal image storing means for storing an orthogonal image corresponding to a tomographic image in a direction orthogonal to a succession direction of the tomographic images;
orthogonal image displaying means for displaying the stored orthogonal image;
index image producing means for producing an index image with a layer thickness corresponding to the number of images to be combined; and
index image displaying means for displaying the produced index image such that the index image is superimposed on the orthogonal image at a position associated with the combined and displayed tomographic image.

4. The image display device according to claim 3, further comprising:
part extracting means for extracting linear parts at the same position from the number of successive tomographic images; and
orthogonal image producing means for placing the extracted linear parts in the succession direction of the tomographic images to produce the orthogonal image,
wherein the orthogonal image storing means stores the produced orthogonal image.

5. The image display device according to any one of claims 2 to 4, wherein the tomographic image storing means stores series data formed of the number of tomographic images taken from a patient at predetermined intervals, the image display device further comprising:
layer thickness calculating means for multiplying the interval by the number of images to be combined, to calculate a layer thickness of the combined tomographic images; and
layer thickness displaying means for displaying the calculated layer thickness as text data.

6. The image display device according to any one of claims 1 to 5, wherein the switching operation means receives an input operation in a plurality of steps, and
the image switching means continuously switches the displayed tomographic images at a speed in a plurality of steps in accordance with an input operation on the switching operation means.

7. The image display device according to any one of claims 1 to 5, wherein the switching operation means receives an input operation in a plurality of steps, and
the image switching means switches the displayed tomographic images by one in accordance with an input operation in a first stage on the switching operation means, and continuously switches the displayed tomographic images at a speed in accordance with an input operation in a second or higher stage.

8. The image display device according to any one of claims 2 to 7, wherein the shot inputting means receives an input operation in a plurality of steps, and
the display combining means increases or decreases the number of images to be combined at a speed in a plurality of steps in accordance with an input operation on the shot inputting means.

9. The image display device according to any one of claims 2 to 7, wherein the shot inputting means receives an input operation in a plurality of steps, and
the display combining means increases or decreases the number of images to be combined by one in accordance with an input operation in a first stage on the shot inputting means, and continuously increases or decreases the number of images to be combined at a speed in accordance with an input operation in a second or higher stage.

10. The image display device according to any one of claims 2 to 5, further comprising:
an input operation apparatus which receives a forward and rearward input operation in a plurality of steps; and
function selecting means operated for causing the input operation apparatus to selectively function as one of the switching operation means and the shot inputting means,
wherein the image switching means continuously switches the displayed tomographic images at a speed in a plurality of steps in accordance with an input operation on the switching operation means, and
the display combining means increases or decreases the number of images to be combined at a speed in a plurality of steps in accordance with an input operation on the shot inputting means.

11. The image display device according to any one of claims 2 to 5, further comprising:
an input operation apparatus which receives a forward and rearward input operation in a plurality of steps; and
function selecting means operated for causing the input operation apparatus to selectively function as one of the switching operation means or the shot inputting means,
wherein the image switching means switches the displayed tomographic images by one in accordance with an input operation in a first stage on the switching operation means, and continuously switches the displayed tomographic images at a speed in accordance with an input operation in a second or higher stage, and
the display combining means increases or decrease the number of images to be combined by one in accordance with an input operation in a first stage on the shot inputting means, and continuously increases or decreases the number of images to be combined at a speed in accordance with an input operation in a second or higher stage.

12. The image display device according to any one of claims 1 to 11, wherein the display combining means superimposes n images of the tomographic images one on another.

13. The image display device according to any one of claims 1 to 11, wherein the display combining means averages n tomographic images.

14. A method of displaying an image in an image display device which stores series data formed of a number of successive tomographic images, displays the stored tomographic images, receives an input operation for switching the tomographic images in a continuous forward or rearward direction, and switches the displayed tomographic images in the successive forward or rearward direction in accordance with the input operation, comprising the step of combining n successive images of the tomographic images and displaying the combined image.

15. A computer program for an image display device which stores series data formed of a number of successive tomographic images, displays the stored tomographic images, receives an input operation for switching the tomographic images in a continuous forward or rearward direction, and switches the displayed tomographic images in the successive forward or rearward direction in accordance with the input operation,
the computer program causing the image display device to combine n successive images of the tomographic images and display the combined image.
